# EUROPEAN PATENT APPLICATION

(11) **EP 2 476 390 A1**
(43) Date of publication of application: **18.07.2012**
(21) Application number: 12000110.2
(22) Date of filing: 10.01.2012
(51) Int. Cl.: A61C 8/00, A61L 27/00

(54) **Dental implant and surface treatment method of dental implant**

(30) Priority: 13.01.2011 JP 2011004444
(71) Applicant: GC Corporation, Tokyo 113-0033 (JP)
(72) Inventor: Takagi, Takamitsu, Tokyo (JP); Yamanaka, Katsuyuki, Tokyo (JP); Noguchi, Tsuyoshi, Tokyo (JP)
(74) Representative: Müller-Boré & Partner Patentanwälte

(57) **Abstract**

A dental implant is made of a mono metal body from inside to outermost surface of the implant, and the implant surface has macro pores having an inner diameter with the level of several tens of µm, micro pores having an inner diameter of 1 to 2 µm, and nano discharge craters having an inner diameter with the level of several tens of nm. A surface treatment method of the implant includes steps of producing macro pores having an inner diameter with the level of several tens of µm by blast processing, producing micro pores having an inner diameter of 1 to 2 µm by acid treatment, and producing nano discharge craters having an inner diameter with the level of several tens of nm by anodic oxidation treatment.

## Description

The present invention relates to a dental implant to be directly embedded in a jawbone to recover an occlusal function, and its surface treatment method.

In recent years, a dental implant treatment has been widely used as one of dental prosthesis treatments. In the dental implant treatment, a dental implant made of titanium or titanium alloy having excellent biocompatibility is embedded in a jawbone of a lost tooth part and substituted for a natural tooth root through the directly bonding to the bone (osseointegration).

An implant body embedded in the jawbone passes through contacting to biological tissues such as blood, fibroblast, osteoblast, etc., then a healing period and finally functions as an artificial tooth root in a state in which the osseointegration is obtained. Therefore, many implants distributed in the current market have modified implant surface from mechanically machined surface of the implant to surface modification, so that the implant body is easily bonded to a bone tissue.

As a conventional method of surface treatment, it has been proposed, i.e., a method for making a roughened surface with micro roughness of 0.5 to 2 µm by combining a blast processing and an acid treatment (for example, refer to Japanese Patent Application Laid-Open No. H3-47264), a method for making a macro surface by anodic oxidation treatment (for example, refer to Japanese Translation of PCT International Application Publication No. 2003-500160), a method for making micro surface and nano surface by depositing calcium phosphate nanocrystals on the surface after an acid treatment (for example, refer to Japanese Translation of PCT International Application Publication No. 2009-515600), and a method for providing micro roughness including fine pores on the implant surface by giving fluorine and/or fluoride on at least a part of the implant surface, wherein the micro roughness includes a root mean square roughness (Rq and/or Sq) of 250nm or less, and/or fine pores each has a pore diameter of 1 µm or less, and a pore depth of 500 nm or less (for example, refer to Japanese Translation of PCT International Application Publication Nos. 2005-533551 and 2005-533552).

The implant surface characteristics according to the conventional art will be summarized below. That is, it has been known that the macro surface with surface roughness with a level of several tens of µm has an effect of increasing a surface area of the implant surface to increase a contact area with biological tissues. Further, it has been known that the micro surface with surface roughness of 1 to 2 µm has an effect, on the surface of the implant, of improving wettability and maintaining capability of blood clot with the direction of fibrin fibers originating from blood, and the like. Furthermore, it has been known that the nano surface with surface roughness with the level of several tens to several hundreds of nm has an effect of increasing cell adhesion strength, the amount of a bone active substance originating from an osteoblast, the amount of calcium deposition, and the like.

However, there are no implants made of a mono metal body entirely from inside of the implant to outermost surface of the implant with a nano surface, and a macro surface or micro surface are formed uniformly on the same implant surface. The implant proposed in aforementioned Japanese Patent Application Laid-Open No. H3-47264 does not include the nano surface, and the implant proposed in aforementioned Japanese Translation of PCT International Application Publication No. 2003-500160 does not include the micro surface and nano surface. Aforementioned Japanese translation of PCT International Application Publication No. 2009-515600 proposes the method for depositing nanocrystals on the micro surface. However, the deposited nano hydroxyapatite crystal may peel off because the crystal is different material of the implant. The methods proposed in aforementioned Japanese Translation of PCT International Applications Publication Nos. 2005-533551 and 2005-533552 are not obtained by the implants with the micro pores and the nano surface uniformly on the entire implant surface.

It is an object of the present invention to provide a dental implant which is made of a mono metal body from inside to outermost surface of the implant, and has a nano surface, and a macro surface or micro surface uniformly formed on the same implant surface.

Namely, the present invention is directed to a dental implant made of the mono metal body from inside to outermost surface of the implant surface. The implant surface has macro pores having an inner diameter with the level of several tens of µm, micro pores having an inner diameter of 1 to 2 µm, and nano discharge craters having an inner diameter with the level of several tens of nm. Or, the implant surface has macro pores having an inner diameter with the level of several tens of µm, and nano discharge craters having an inner diameter with the level of several tens of nm. Or, the implant surface has micro pores having an inner diameter of 1 to 2 µm, and nano discharge craters having an inner diameter with the level of several tens of nm.

Further, the present invention relates to a surface treatment method of the implant which is made of a the mono metal body from inside to outermost surface of the implant surface. The method includes a step for producing macro pores having an inner diameter with the level of several tens of µm by blast processing, a step for producing micro pores having an inner diameter of 1 to 2 µm by acid treatment, and a step for producing nano discharge craters having an inner diameter with the level of several tens of nm by anodic oxidation treatment. Or, the surface treatment method includes a step for producing macro pores having an inner diameter with the level of several tens of µm by blast processing, and a step for producing nano discharge craters having an inner diameter with the level of several tens of nm by anodic oxidation treatment. Or, the surface treatment method includes a step for producing micro pores having an inner diameter of 1 to 2 µm by acid treatment, and a step for producing nano discharge craters having an inner diameter with the level of several tens of nm by anodic oxidation treatment.

According to the implant and the surface treatment method of the implant of the present invention, each characteristic on the implant surface of a contact surface area, wettability, maintaining capability of blood clot, cell adhesion strength, a bone active substance originated by osteoblasts, the amount of calcium deposition, and the like, can be improved.
Fig. 1 is a SEM picture showing macro pores formed on the implant surface of one exemplary embodiment according to the present invention.
Fig. 2 is a SEM picture showing micro pores formed on the implant surface of one exemplary embodiment according to the present invention.
Fig. 3 is a SEM picture showing nano discharge craters formed on the implant surface of one exemplary embodiment according to the present invention.

A first embodiment of the present invention is directed to a dental implant made of a mono metal body from inside to outermost surface of the implant, wherein the implant surface has macro pores having an inner diameter with the level of several tens of µm, micro pores having an inner diameter of 1 to 2 µm, and nano discharge craters having an inner diameter with the level of several tens of nm.

According to the first embodiment, the implant surface having the macro pores, the micro pores, and the nano discharge craters can be obtained, and each characteristic of a contact surface area, wettability, maintaining capability of blood clot, cell adhesion strength, a bone active substance originated by osteoblasts, the amount of calcium deposition, and the like, can be improved.

The second embodiment is directed to a dental implant made of a mono metal body from inside to outermost surface of the implant, wherein the implant surface has macro pores having an inner diameter with the level of several tens of µm, and nano discharge craters having an inner diameter with the level of several tens of nm.

According to the second embodiment, the implant surface having the macro pores and the nano discharge craters can be obtained, and each characteristic of a contact surface area, cell adhesion strength, a bone active substance originated by osteoblasts, the amount of calcium deposition, and the like, can be improved.

The third embodiment of the present invention is directed to a dental implant made of a mono metal body from inside to outermost surface of the implant, wherein the implant surface has micro pores having an inner diameter of 1 to 2 µm, and having nano discharge craters having an inner diameter with the level of several tens of nm.

According to the third embodiment, the implant surface having the micro pores and nano discharge craters can be obtained, and each characteristic of wettability, maintaining capability of blood clot, cell adhesion strength, a bone active substance originated by osteoblasts, the amount of calcium deposition, and the like, can be improved.

It is preferable in the embodiments of the present invention that the nano discharge craters are of 10 nm or more to less than 100 nm. If the nano discharge craters are of within the aforementioned range, the effect of increasing cell adhesion strength, a bone active substance originated by osteoblasts, the amount of calcium deposition, and the like, can be more improved.

The dental implant according to the present invention is made of a mono metal body from inside to outermost surface of the implant. The metal body is preferably one to four kinds of pure titanium (Grade 1 to 4), a Ti-6Al-4V alloy, or a titanium based alloy including one, two, or three kinds of metal selected from Nb, Zr, Ta, Mo, Sn, and Al.

The first embodiment of the surface treatment method of a dental implant according to the present invention, wherein the implant is made of a mono metal body from inside to outermost surface of the implant, includes a step for producing macro pores having an inner diameter with the level of several tens of µm by blast processing, a step for producing micro pores having an inner diameter of 1 to 2 µm by acid treatment, and a step for producing nano discharge craters having an inner diameter with the level of several tens of nm by anodic oxidation treatment.

According to the method of the present invention, the implant surface having the macro pores, the micro pores, and the nano discharge craters can be obtained, and the implant, which can improve each characteristic of a contact surface area, wettability, maintaining capability of blood clot, cell adhesion strength, a bone active substance originated by osteoblasts, the amount of calcium deposition, and the like, can be obtained.

The second embodiment of the surface treatment method of a dental implant according to the present invention, wherein the implant is made of a mono metal body from inside to outermost surface of the implant, includes a step for producing macro pores having an inner diameter with the level of several tens of µm by blast processing and a step for producing nano discharge craters having an inner diameter with the level of several tens of nm by anodic oxidation treatment.

According to the method of the present invention, the implant surface having the macro pores and the nano discharge craters can be obtained, and the implant, which can improve each characteristic of a contact surface area, wettability, maintaining capability of blood clot, cell adhesion strength, a bone active substance originated by osteoblasts, the amount of calcium deposition, and the like, can be obtained.

The third embodiment of the surface treatment method of a dental implant according to the present invention, wherein the implant is made of a mono metal body from inside to outermost surface of the implant, includes a step for producing micro pores having an inner diameter of 1 to 2 µm by acid treatment, and a step for producing nano discharge craters having an inner diameter with the level of several tens of nm by anodic oxidation treatment.

According to the method of the present invention, the implant surface having the micro pores and the nano discharge craters can be obtained, and the implant, which can improve each characteristic of a contact surface area, wettability, maintaining capability of blood clot, cell adhesion strength, a bone active substance originated by osteoblasts, the amount of calcium deposition, and the like, can be obtained.

In the methods of the present invention, it is preferable that blast particles used in the blast processing are particles made of one or more kinds of materials selected from alumina, magnesia, titania, iron, titanium, and zirconia.

In the methods of the present invention, it is preferable that acid used for the acid treatment is an aqueous solution of an acid selected from hydrochloric acid, sulfuric acid, phosphoric acid, lactic acid, fluoric acid, and hydrogen peroxide, or is a mixture of these acids.

In the methods of the present invention, it is preferable that applied voltage in the anodic oxidation treatment is 10 to 150V.

In the methods of the present invention, it is preferable that a treatment liquid used in the anodic oxidation treatment is one or more kinds selected from an aqueous solution selected from phosphoric acid, sulfuric acid, and aluminum sulfate, a hydrogen peroxide solution, and a mixture of these substances.

### [Examples]

Test pieces (a material: pure titanium of JIS type 4 and Ti-6Al-4V alloy) with the shape of a dental implant, which were made of a mono metal body from inside to outermost surface of the implant, was given macro pores, micro pores, or both of them under each condition shown in Table 1 below. In addition, the step for producing the macro pores having an inner diameter with the level of several tens of µm was carried out by blast processing, before the process to produce the micro pores with an inner diameter of 1 to 2 µm.
HF: Hydrogen fluoride (an aqueous solution of 2% by weight)
HCl-H₂SO₄: Hydrochloric acid / Sulfuric acid (H₂SO₄: HCl: Water = 1 : 2 : 1, by weight ratio)

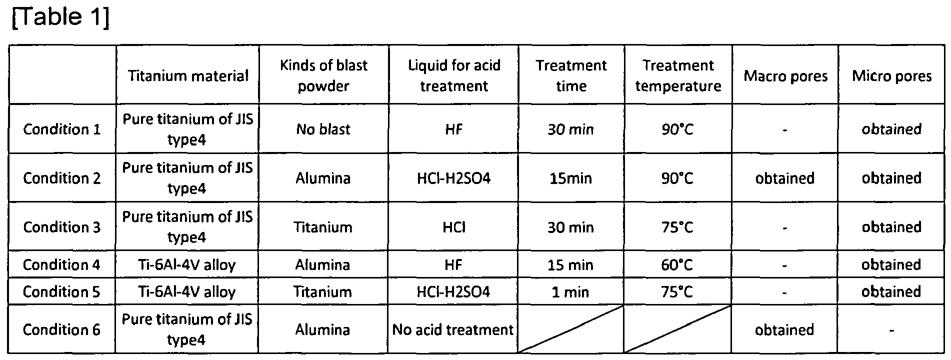

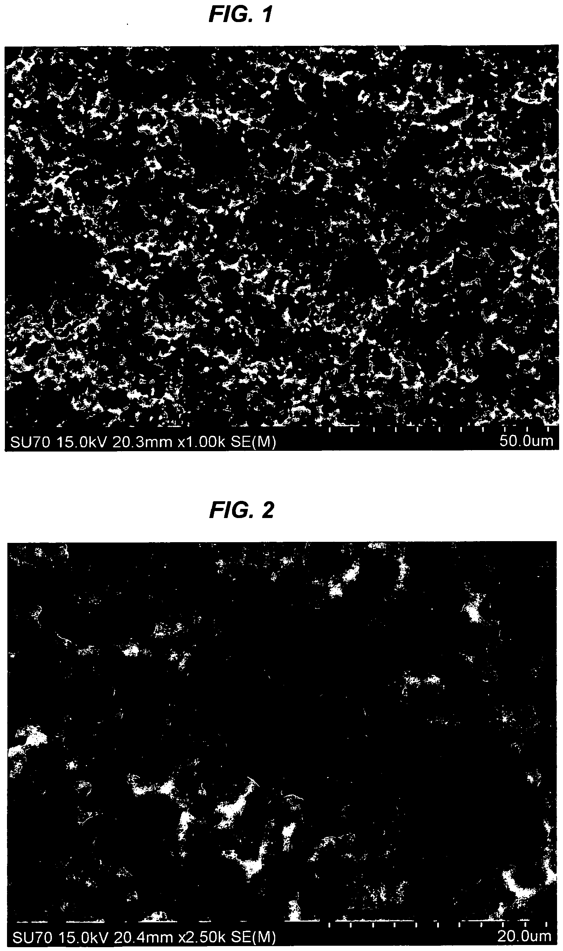

The anodic oxidation treatment was carried out by applying voltage of 110V to the above sample in 10% H₃PO₄ solution, so that nano discharge craters were given. SEM pictures of the implant surface produced under the condition 2 are shown in Figs. 1 to 3.

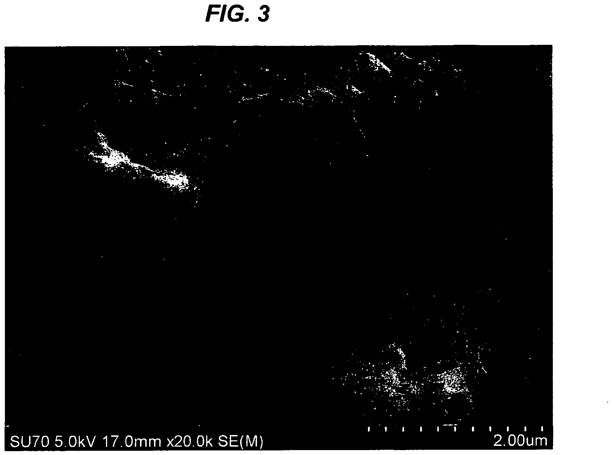

It was confirmed that the implant surface produced under the condition 2 had the macro pores with the level of several tens of µm as shown in Fig. 1, the micro pores having an inner diameter of 1 to 2 µm as shown in Fig. 2, and the nano discharge craters having an inner diameter with the level of several tens of nm as shown in Fig. 3.

The samples under the conditions 1 to 6 shown in Table 1 were subjected to the anodic oxidation treatment, and the acquisition states of each of the macro pores, the micro pores, and the nano discharge craters were investigated by SEM observation. These results were shown in Table 2.

**[Table 2]**

| | *Macro pores* | *Micro pores* | Nano discharge traces |
|---|---|---|---|
| Condition 1 | - | obtained | obtained |
| Condition 2 | obtained | obtained | obtained |
| Condition 3 | - | obtained | obtained |
| Condition 4 | - | obtained | obtained |
| Condition 5 | - | obtained | obtained |
| Condition 6 | obtained | - | obtained |

Under the conditions 1, and 3 to 5, the implant surfaces having the micro pores and the nano discharge craters could be obtained. Under the condition 6, the implant surface having the macro pores and the nano discharge craters could be obtained. Under the condition 2, the implant surface having the macro pores, the micro pores, and the nano discharge craters could be obtained.

## Claims

1. A dental implant made of a mono metal body from inside to outermost surface of the implant,
wherein the implant surface has macro pores having an inner diameter with the level of several tens of µm, micro pores having an inner diameter of 1 to 2 µm, and nano discharge craters having an inner diameter with the level of several tens of nm.

2. A dental implant made of a mono metal body from inside to outermost surface of the implant,
wherein the implant surface has macro pores having an inner diameter with the level of several tens of µm, and nano discharge craters having an inner diameter with the level of several tens of nm.

3. A dental implant made of a mono metal body from inside to outermost surface of the implant,
wherein the implant surface has micro pores having an inner diameter of 1 to 2 µm, and nano discharge craters having an inner diameter with the level of several tens of nm.

4. A dental implant according to any of claims 1 to 3, wherein the nano discharge craters are of 10 nm or more to less than 100 nm.

5. A dental implant according to any one of claims 1 to 3, wherein the metal body is a pure titanium of Grade 1 to 4, a Ti-6Al-4V alloy, or a titanium based alloy including one, two, or three kinds of metal selected from Nb, Zr, Ta, Mo, Sn, and Al.

6. A surface treatment method of a dental implant made of a mono metal body from inside to outermost surface of the implant, the method comprising:
a step for producing macro pores having an inner diameter with the level of several tens of µm by blast processing;
a step for producing micro pores having an inner diameter of 1 to 2 µm by acid treatment; and
a step for producing nano discharge craters having an inner diameter with the level of several tens of nm by anodic oxidation treatment.

7. A surface treatment method of a dental implant made of a mono metal body from inside to outermost surface of the implant, the method comprising:
a step for producing macro pores having an inner diameter with the level of several tens of µm by blast processing; and
a step for producing nano discharge craters having an inner diameter with the level of several tens of nm by anodic oxidation treatment.

8. A surface treatment method of a dental implant made of a mono metal body from inside to outermost surface of the implant, the method comprising:
a step for producing micro pores having an inner diameter of 1 to 2 µm by acid treatment; and
a step for producing nano discharge craters having an inner diameter with the level of several tens of nm by anodic oxidation treatment.

9. A surface treatment method according to claim 6 or 7, wherein blast particles used for the blast processing are particles made of one or more kinds selected from alumina, magnesia, titania, iron, titanium, and zirconia.

10. A surface treatment method according to claim 6 or 8, wherein acid used for the acid treatment is an aqueous solution of an acid selected from hydrochloric acid, sulfuric acid, phosphoric acid, lactic acid, fluoric acid, and hydrogen peroxide, or is a mixture of these acids.

11. A surface treatment method according to any one of claims 6 - 8, wherein applied voltage in the anodic oxidation treatment is 10 to 150V.

12. A surface treatment method according any one of claims 6 - 8, wherein a treatment liquid used in the anodic oxidation treatment is one or more kinds selected from an aqueous solution selected from phosphoric acid, sulfuric acid, and aluminum sulfate, a hydrogen peroxide solution, or a mixture of these substances.
